# EUROPEAN PATENT APPLICATION

(11) **EP 1 527 798 A2**
(43) Date of publication of application: **04.05.2005**
(21) Application number: 05000735.0
(22) Date of filing: 08.09.1995
(51) Int. Cl.: A61N 5/06

(54) **Diffusive tip assembly**

(30) Priority: 09.09.1994 US 303605; 06.06.1995 US 467414; 06.06.1995 US 468568; 06.06.1995 US 471744
(62) Divisional of application: 04029580.0
(71) Applicant: Cardiofocus, Inc., West Yarmouth, MA 02673 (US)
(72) Inventor: Sinofsky, Edward L., Dennis, MA 02638 (US); Baxter, Loncoln S., Centerville, MA 02632 (US); Farr, Norman, Monument Beach, MA 02553 (US)
(74) Representative: Strehl Schübel-Hopf & Partner

(57) **Abstract**

Optically transmissive fiber tip assemblies having radiation-scattering particles (**24**) incorporated therein are disclosed for use in phototherapy. In one embodiment, diffusers with reflective end caps are disclosed. As radiation propagates through the fiber tip, a portion of the radiation is scattered in a cylindrical (or partially cylindrical) pattern along the length of the fiber tip. Radiation which is not scattered during this initial pass through the tip can be reflected by at least one surface (**28**) of the assembly and returned through the tip. During this second pass, the remaining radiation (or at least a major portion of this returning radiation) again encounters the scatterers which provide further radial diffusion of the radiation. The scattering medium (**22**) and the reflective end cap can interact to provide a substantially uniform (or other predefined) axial distribution of laser radiation over the length of the tip apparatus. In another embodiment, diffusive loop assemblies are disclosed for diffusing radiation from an optical fiber. Disposed sheaths are also disclosed, as are integral stopper devices which limit the penetration of the apparatus. The devices of the invention are useful for various medical purposes as well as the sterilization of medical instruments.

## Description

### Background of the Invention

The technical field of this invention is phototherapy and, in particular, methods and devices which employ optical fibers or other flexible light waveguides to deliver radiation to a targeted site.

Fiber optic phototherapy is a increasing popular modality for the diagnosis and/or treatment of a wide variety of diseases. For example, in surgery, infrared laser radiation will often be delivered to a surgical site via a hand-held instrument incorporating an optically transmissive fiber in order to coagulate blood or cauterize tissue. Similar fiber optic delivery systems have been proposed for endoscopic or catheter-based instruments to deliver therapeutic radiation to a body lumen or cavity. U.S. Patent No. 4, 336,809 (Clark) and U.S. Reissue Patent No. RE 34,544 (Spears) disclose that hematoporphyrin dyes and the like selectively accumulate in tumorous tissue and such accumulations can be detected by a characteristic fluorescence under irradiation with blue light. These patents further teach that cancerous tissue that has taken up the dye can be preferentially destroyed by radiation (typically high intensity red light) that is absorbed by the dye molecules during phototherapy.

Others have proposed the use of fiber-delivered radiation to treat artherosclerotic disease. For example, U.S. Patent 4,878,492 (Sinofsky *et al.)* discloses the use of infrared radiation to heat blood vessel walls during balloon angioplasty in order to fuse the endothelial lining of the blood vessel and seal the surface. Another application of fiber-delivered radiation is disclosed in U.S. Patent 5,053,033 (Clarke) which teaches that restenosis following angioplasty can be inhibited by application of UV radiation to the angioplasty site to kill smooth muscle cells which would otherwise proliferate in response to angioplasty-induced injuries to blood vessel walls.

Nonetheless, a number of problems limit the expanded use of fiber-optic phototherapy. Typically, an optical fiber emits light from only its end face. Thus, the emitted light tends to be focused or at best divergent in a conical pattern and, therefore, exposes only a small region directly in front of the fiber's distal end. The small exposure area limits the power available for phototherapy since overheating of the target tissue must often be avoided.

Although "sideways-emitting" fibers have been proposed to permit greater flexibility in phototherapy, this approach still does not allow uniform irradiation of large volumes of tissue and can also be ill-suited for applications where circumferential uniformity is desired. Because sideways-emitting fibers expose limited regions, they do little to alleviate the problem of "hot spots" which limit the intensity of radiation which can be delivered via the fiber to the treatment site.

Others have proposed diffusive tips for optical fibers to enlarge the region which can be irradiated and/or reduce the potential for overexposure. However, diffusive tips have not been satisfactory for many therapeutic purposes because of their complexity of manufacture and/or because the radiation may not be scattered uniformly enough to alleviate the problem of "hot spots." Prior art diffusive tip structures have not be capable of delivering high power radiation, e.g., on the order of ten watts or more, to facilitate photocoagulation therapy or the like.

There exists a need for better apparatus for fiber-optic phototherapy. In particular, diffusive fiber tip assemblies which can provide circumferential (or large angle) exposure regions in radial directions (e.g., sideways) relative to the fiber axis without hot spots would satisfy a long-felt need in the art. Moreover, diffusive assemblies which illuminate or irradiate an azimuthal angle of less than 360° would meet a particularly important need in the field of minimally-invasive, phototherapeutic surgery. Similarly, diffusive assemblies that provide graded or broadly-cast exposure patterns, or otherwise predefined patterns of light distribution would also meet particular needs. In addition, diffusive fiber tip assemblies which can extend the longitudinal extent of irradiation and provide greater flexibility during use would also satisfy a need in phototherapy.

In another application, phototherapeutic instruments can be employed to treat electrical arrhythmia of the heart. In such applications, a catheter having a fiber optic component is fed via a major artery into a patient's heart. Once inside the heart, a catheter senses electrical impulses with electrical contacts on its outer sheath or other catheter elements in order to locate the source of arrhythmia. Once located, the phototherapeutic component is activated to "ablate" a portion of the inner heart wall. By coagulating the tissue in the vicinity of the arrhythmia source, the likelihood that the patent's heart will continue to experience arrhythmia is thus reduced.

In other applications, laser radiation can be used in conjunction with a similar catheter instrument inside a patient's heart to increase blood flow to oxygen starved regions of the heart muscle. In such procedures, the laser radiation is used to form small holes into the heart muscle so that the oxygen-depleted tissue is bathed with blood from the ventricular cavity.

In all of these applications, there is the potential for damage to the patient's internal organs, especially the heart, if the light-emitting fiber is inserted too far into the patient's tissue. Particularly, in the case of the heart muscle, perforation of the heart wall can have very dangerous effects.

Accordingly, there exists a need for better apparatus for fiber-optic phototherapy. In particular, devices that can "stop" an optical fiber from perforating a patient's organs would meet a particularly important need in the field of minimally-invasive phototherapeutic surgery. Moreover, a device that can help stabilize the phototherapeutic instrument in operation (such as within the chambers of a rapidly beating heart) would also be particularly useful.

In yet another application, phototherapeutic devices can be useful in sterilizing medical instrument lumens. For example, endoscopic instruments are complex and expensive medical devices, which permit the clinician to view the internal organs and structures of a patient's body. These instruments are typically reused and, therefore, must be sterilized after each use. Moreover, because many endoscopes are used repeatedly throughout a day, sterilization of the instruments must be performed rapidly in busy clinics.

Conventionally, endoscopes are sterilized using a chemical bath. The internal lumens of the instrument will either be soaked in a sterilizing liquid or flushed with the sterilizing liquid.

Unfortunately, conventional techniques can sometimes be less than totally effective. The sterilizing liquid may not penetrate the entire lumen or may not be sufficiently strong to achieve the desired antimicrobial effect. Moreover, the endoscope lumen may have accumulated cellular debris that cannot be simply flushed out and such debris may harbor microbes that are not destroyed in the cleaning process.

Accordingly, there exists a need for better methods and devices for sterilizing the inner lumens of endoscopic instruments. Methods and devices which could ensure more effective anti-microbial action and/or permit more rapid sterilization of instrument lumens, would satisfy a long-felt need in the art.

### Summary of the Invention

Methods and apparatus are disclosed for diffusing radiation from a optical fiber to provide a larger exposure area for phototherapy. The methods and apparatus are particularly useful as part of fiber optic-based medical laser systems. The present invention can further provide substantially uniform or otherwise predefined patterns of energy distribution to a major portion of the exposure area. The invention is especially useful in constructing and implementing circumferential, broadly-cast, graded and/or sideways-emitting diffusive tip assemblies for optical fibers to direct laser radiation in one or more radially outward patterns relative to the fiber's axis. As used herein the term "optical fiber" is intended to encompass optically transmissive waveguides of various shapes and sizes.

In one aspect of the invention, an optical transmissive fiber tip structure is disclosed having a radiation-scattering particles and a reflective end. As radiation propagates through the fiber tip, the radiation is scattered. Each time the radiation encounters a scatterer particle, it is deflected until some of the radiation exceeds the critical angle for internal reflection and exits the tip Radiation which is not emitted during this initial pass through the tip is reflected by at least one end surface and returned through the tip. During this second pass, the remaining radiation (or at least a major portion of this returning radiation) again encounters the scatterers which provide further radial diffusion of the radiation.

In one embodiment, a diffusive tip assembly is disclosed for diffusing radiation from an optical fiber. The tip assembly includes a light transmissive, tubular housing alignable with, and adapted to receive, the distal end of the fiber and serve as a waveguide for light propagating through the fiber. The assembly further includes a reflective end cap and a light scattering medium disposed therein such that light propagating through said fiber enters the scattering medium and a portion of the light escapes outward through the housing, and another portion passes through the scattering medium and is reflected by the end cap for retransmission through said scattering medium.

The reflective surfaces of the apparatus can also be modified to effect non-cylindrical or non-spherical exposure patterns. Reflective structures are disclosed which control the azimuthal extent of the light emitted from the tip. These techniques and structures permit, for example, 270 degrees, 180 degrees or even smaller angles of azimuthal exposure. The term "large angle exposure" is used herein to describe partially cylindrical (or partially spherical) exposure patterns having a azimuthal angle of more than about 90 degrees.

In another aspect of the invention, the amount of incorporated scatterers and/or the length of the diffusive tip can be controlled such the diffusion of the radiation beam during the initial and reflected paths are complementary. By proper choice of such parameters, the cumulative energy density or fluence along at least a portion of the length of fiber tip can be rendered uniform. The invention thus provides a mechanism for uniform cylindrical irradiation of biological structures and the like.

In yet another embodiment of the invention, the amount of incorporated scatterers can be varied to create a graded or otherwise varied pattern of exposure. For example, more scatterers can be incorporated into a distal portion of the diffuser assembly to create a progressively increasing exposure pattern. Alternatively, a transparent teflon rod can transmit to a distal mirror to create an increasing intensity distal to the fiber.

In a further aspect of the invention, bundling techniques and configurations are disclosed for extending the axial extent of diffusive irradiation and/or for permitting selective activation of fibers or fiber subsets to effect site-specific phototherapy of regions or sectors of a patient's tissue in the vicinity of the optical fiber tip. Such bundled systems can also be used to deliver two or more different wavelengths of radiation to the treatment site and thereby provide synergistic effects from multiple wavelengths of therapy, or permit diagnostic and therapeutic radiation of different wavelengths to be delivered in a single procedure.

In yet another aspect of the invention, novel materials and structures are disclosed for diffusive tip assemblies to alleviate or reduce the potential for contact-adhesion between the tip and nearby tissue segments. This aspect of the invention is particularly useful in connection with endoscopic and/or catheter-based phototherapy to ensure that the diffusive tip does not bond accidentally to the body lumen or blood vessel wall during procedures. In one embodiment, fluoropolymer materials, such as Teflon® materials and the like, are disclosed as preferred materials for the tip enclosure and/or the outer cladding or coating to inhibit contact-adhesion between the tip assembly and biological tissue during procedures. Most preferably, the Teflon® material is a Teflon® FEP material (a polyperfluoroethylene-propylene copolymer). Other Teflon@ materials such as Teflon® PFA (a polytetrafluoroethylene polymer with perfluoroalkoxy side chains) and Teflon® PTFE (polytetrafluoroethylene) also can be useful in certain applications.

In a further aspect of the invention, novel scatterer structures are disclosed which permit the diffusion of ultraviolet (UV) and infrared (IR) radiation with higher efficiency than prior art structures. Liquid-filled scattering assemblies and, in particular, structures employing deuterium oxide and other heavy water solutions are disclosed which transmit IR light with low losses and minimal tip heating. Distilled water suspensions of scatterers are disclosed for UV light delivery.

In yet a further aspect of the invention, novel treatment protocols are disclosed for minimally invasive phototherapeutic surgery. For example, protocols for the treatment of prostate cancer and similar diseases are disclosed in which a diffusive tip assembly is placed in the vicinity of the cancerous organ or body structure and diffuse light is used to heat and selectively destroy cancerous or dysplastic tissue. In addition, the present invention can be use for the closure of body ducts and/or the reconstruction of competent junctures between ducts or valves that have been malformed or damaged. Moreover, photoactivation of pharmacological agents, implanted structures, or suture materials can all be advantageously effected with the diffusive assemblies of the present invention. In yet a further application of the invention, the phototherapeutic devices disclosed herein can be used to sterilize medical instruments.

In another aspect of the invention, a plurality of optically-transmissive fiber tip assemblies are disclosed to act as diffusers. The two or more fiber tip assemblies are deployed as loops which create a broadly cast and relatively uniform illumination pattern. By "looping" or "folding" the fibers, a plurality of fibers can be deployed in conjunction with one another to create geometric exposure patterns with increased energy density while still avoiding "hot spots."

Such loop diffusers can be incorporated into an endoscopic instrument or catheter. The diffusive elements can be initially deployed in a retracted position (largely within the body of the instrument) and then redeployed with the help of a control wire or the like in an expanded configuration. Thus, the two or more loops in the expanded configuration can create a "globe-like" diffuser assembly, or, if further extended, the loops can form a "heart shaped configuration. The invention thus permits a relatively small instrument to be enlarged to project a wide exposure area.

The individual loops each include a light transmissive, tubular housing aligned with, or adapted to receive, the distal end of a fiber and serve as a wave guide for light propagating through the fiber. In one embodiment, the tubular housing can be a hollow tube filled with a scattering medium and an optical fiber is joined to each end. Light propagating through the fibers will enter opposite ends of the housing and be scattered before reaching the other end In another embodiment, the assembly can be attached to a single fiber and further includes an end cap and a light scattering medium disposed within the housing such that light propagating through the fiber enters the scattering medium and a portion of the light escapes outward through the housing. In the one embodiment of the capped assembly, the end cap is a simple stopper and substantially all of the light eventually is scattered before it reaches the stopper. In another embodiment, the end cap can include a reflective surface such that as the light propagates through the fiber some of it is initially scattered by the scattering medium and exits radially, while another portion passes through the scattering medium and is reflected by the end cap for transmission through the scattering medium again.

In another aspect of the invention, the amount of incorporated scatterers and/or the length of the diffusive loop can be controlled such that the diffusion of the radiation beam during the initial and the reflected paths are complementary. By proper choice of parameters, the cumulative energy density or fluence along at least a portion of the length of the fiber tip can be rendered uniform.

In yet another aspect of the invention, disposable sheaths are disclosed for use in conjunction with the diffuser assemblies. The outer sheath surrounds the entire optical transmission apparatus and ensures that the radiation-generating components do not come into direct contact with the patient's body structures. This permits reuse of the instrument. Only the sheath surrounding the apparatus needs to be disposed after each use.

Phototherapeutic instruments are also disclosed having integral stopper devices which limit the penetration of an optical fiber tip. In one preferred embodiment, a fluted outer sheath is disposed about an internal optical fiber. The fluted sheath is configured to fold into an expanded form during penetration into a patient's tissue. As the sheath is forced back and expands, the optically-transmissive assembly presents a much larger cross-sectional area which prevents penetration of the instrument beyond a pre-determined desirable distance.

The invention is particularly useful in limiting an optically-transmissive fiber's penetration and, thereby, reduce the possibility of perforation of a body lumen or organ. The invention is particularly useful in placing a "ablative" laser radiation device into the ventricle of the heart when performing arrhythmia-correcting laser ablative procedures or when revascularizing the heart percutaneously. In these types of procedures, the surgeon seeks to partially penetrate the heart muscle while not fully perforating the heart wall. The stopper devices of the present invention limit penetration and stabilized the optically-transmissive tip during phototherapy.

The structures disclosed herein represent a substantial step forward in the delivery of therapeutic radiation to remote treatment sites. The diffusive assembly designs of the present invention permit the delivery of radiation at power levels on the order of tens of Watts or more. In fact, diffusive tip assemblies have been successfully constructed to deliver over 100 Watts of power in a diffuse pattern to a treatment site, allowing the clinician to perform therapy rapidly and uniformly to a large volume of tissue.

Methods and devices are further disclosed for sterilization of endoscopic instrument lumens. Diffuse ultraviolet radiation is employed to sterilize the inner surfaces of the instrument lumen. The ultraviolet radiation can be delivered via one or more optical fibers having a light-diffusing assembly coupled thereto. The instrument operates by delivering cytotoxic radiation to the inner lumen surface to sterilize any biological agents which may be present within the instrument lumen.

In this aspect of the invention a lumen sterilizing apparatus is disclosed having a light transmitting fiber which is capable of transmitting ultraviolet radiation. The apparatus further includes a diffuser means, coupled to the fiber for diffusing ultraviolet radiation from the fiber. The fiber and diffuser are sufficiently small so as to fit within an endoscope lumen. The apparatus further includes an irradiation means for generating ultraviolet radiation and for coupling the radiation to the fiber.

The sterilizing ultraviolet radiation preferably ranges from about 400 to about 200 nanometers in wavelength and, more preferably, from about 300 to 220 nanometers, and most preferably, from about 280 to 240 nanometers. Such radiation can be obtained from a laser source, such as an Argon ion laser or an excimer laser (such as a Xenon Chloride excimer laser). Alternatively, a solid state laser can be used in conjunction with frequency modifying element. For example, an infrared radiation source can be used in conjunction with two frequency-doubling crystals, which cooperate to yield a frequency-quadrupled radiation beam in the ultraviolet spectrum. In yet another alternative embodiment, a simple ultraviolet flash lamp can be employed as the light source and coupled to the optical fiber.

The optical fiber can be any conventional optic transmission element including, for example, fused silica. As used herein, the term "optical fiber" is intended to encompass optically transmissive wave guides of various shapes and sizes.

In one embodiment, a diffusing tip can be employed in conjunction with the optical fiber to deliver diffuse cytotoxic radiation to the inner lumen. The diffusive fiber tip structure can be formed by radiation-scattering particles carried in a suitable transmission medium Alternatively, the diffusing tip can be constructed from a tubular element filled with any suitable medium that diffuses light without the need for particulate scatterers. For example, a longer tube filled with water or acetic acid can also serve as the scattering medium. In this embodiment it may not be necessary to move the diffusing tip. Instead, the apparatus can be used to sterilize a substantial portion, or the entire length, of the lumen all at once.

In another aspect of the invention, novel materials and structures are disclosed for diffusive tip assemblies which further alleviate or reduce the potential for contact-adhesion between the tip and the nearby lumen wall. This aspect of the invention is particularly useful to ensure that the diffusive tip does not accidentally bond to the instrument lumen or debris within the lumen. In one embodiment, fluoropolymer materials, such as Teflon® materials and the like are disclosed as preferred materials for the tip enclosure because of their low contact-adhesion characteristics, deep ultraviolet transmissivity and low refractive index.

In yet another aspect of the invention, disposable sheaths are disclosed for use in conjunction with the ultraviolet sterilizing fiber and diffuser assembly. The outer sheath surrounds the entire optical transmission apparatus and ensures that the radiation-generating components do not come into direct contact with the instrument lumen or the debris which may be present in such lumen. This permits reuse not only of the endoscope repeatedly by a clinician, but also reuse of the sterilizing instrument. Only the sheath surrounding the sterilization apparatus need to be disposed after each use. Alternatively, a disposable sheath/diffuser can be used in conjunction with a reusable fiber. Thus, a disposable sheath filled with a light scattering medium can be fitted to a reusable fiber and then used to perform instrument sterilization. When the procedure is completed, the sheath and the scattering medium inside can then be discarded.

In a further aspect of the invention, methods are disclosed for performing instrument sterilization. These methods typically involve the placement of an ultraviolet radiation-diffusing assembly within the endoscopic instrument lumen and then the pulling of the sterilization apparatus through the lumen such that the entire inner surface is bathed with cytotoxic radiation. The method can further include the use of a disposable outer sheath which surrounds the sterilization apparatus while it is being pulled through the lumen and then is discarded after the sterilization procedure is completed.

The terms "endoscopic instrument" and "endoscope" are used herein to describe a general class of instrument useful in viewing internal body structures or performing operations within a patient's body, including cystoscopes, tracheoscopes, culpascopes, proctoscopes, laprascopes, catheters, arthroscopes, other endoscopes and the like.

The invention will next be described in connection with certain preferred embodiments. However, it should be clear that various changes and modifications can be made by those skilled in the art without departing from the spirit and scope of the invention.

### Brief Description of the Drawings

The invention may be more fully understood from the following description when read together with the accompanying drawings in which:
FIG. **1** is a cross-sectional illustration of a phototherapeutic apparatus incorporating an optical fiber and a diffusive tip assembly in accordance with the present invention;
FIG. **2** is another cross-sectional illustration of a phototherapeutic apparatus in accordance with the present invention incorporating a plurality of optical fibers and a diffusive tip assembly;
FIG. **2A** is a cross-sectional view of the optical fiber diffusive tip assembly of FIG. **2** taken along the line A-A of FIG. **2**;
FIG. **3** is another cross-sectional illustration of a phototherapeutic apparatus in accordance with the present invention incorporating a plurality of optical fibers and a diffusive tip assembly in which the fibers have different terminal points within the assembly;
FIG. **3A** is perspective view of the end portions of the optical fibers of FIG. **3**;
FIG. **4** is another cross-sectional illustration of a phototherapeutic apparatus in accordance with the present invention incorporating a multilayer laminated scatterer tube element;
FIG. **5** is another cross-sectional illustration of a phototherapeutic apparatus in accordance with the present invention incorporating a longitudinal reflector to provide azimuthal selectivity in a diffusive tip assembly;
FIG. **5A** is a cross-sectional view of the optical fiber diffusive tip assembly of FIG. **5** taken along the line **A-A** of FIG. **5**;
FIG. **6A** is a cross-sectional illustration of an alternative reflector design useful in diffusive tip assemblies according to the invention;
FIG. **6B** is a cross-sectional illustration of another alternative reflector design useful in diffusive tip assemblies according to the invention;
FIGS. **7A**, **7B**, and **7C** are graphs illustrating the relationship between relative intensity and axial distance from the fiber end face for various scatterer loading concentrations;
FIG. **8** is graph of intensity versus axial position for various mirror placements in diffusive tip assemblies according to the invention;
FIG. **9** is graph of intensity versus axial position for an actual diffusive tip assembly according to the invention;
FIG. **10** is a graph of azimuthal intensity distribution of two diffusive tip assemblies according to the invention, one providing a cylindrical exposure pattern and the other providing a semi-cylindrical pattern;
FIG. **11** is graph of the transmission spectrum of Teflon® FEP illustrating the relationship between transmissivity and wavelength;
FIG. **12** is a cross-sectional illustration of another phototherapeutic apparatus according to the invention having two chambers filled with different scattering media to effect an increasing diffusion pattern;
FIG. **13** is a is a schematic perspective view of a loop diffuser in accordance with the present invention;
FIG. **14A** is a side view of a loop diffuser in which the diffusive elements are fully retracted;
FIG. **14B** is a side view similar to that of FIG. **14A** in which the loop diffuser elements are partially deployed;
FIG. **14C** is a further side view of the instrument in which the loop diffuser elements are fully deployed;
FIG. **14D** is a further side view of the instrument in which the loop diffuser elements are fully deployed with the control wire partially retracted to effect a "heart-shaped" diffuser;
FIG. **15A** is a cross-sectional view of an optical fiber diffusive tip assembly for use in the apparatus of FIG. **13**.
FIG. **15B** is a graph of intensity vs. axial distance for the loop diffuser of FIG. **15A**;
FIG. **16** is a cross-sectional view of another optical fiber diffusive tip assembly for use in the apparatus of FIG. **13**;
FIG. **17** is a schematic view of the use of present invention as part of an endoscopic system;
FIG. **18** is a further cross-sectional view of an optical fiber and diffusive tip assembly in accordance with the present invention, further employing a disposable outer sheath;
Fig. **19** a schematic, perspective field of the distal end of a phototherapeutic apparatus and integral stopper device in accordance with the present invention;
Fig. **20** is a cross-sectional illustration of the phototherapeutic apparatus of Fig. **19**;
Fig. **21** is a schematic view of the present invention as part of a catheter or endoscopic system;
Fig. **22A** illustrates the phototherapeutic apparatus of the present invention deployed in an initial position prior to contacting the surface of a body organ or lumen;
Fig **22B** is a further illustration of the apparatus of Fig **22A** after initial penetration of body tissues;
Fig. **22C** is a further schematic illustration of the penetration of the phototherapeutic apparatus of Fig **22A** in which the stopper mechanism is partially deployed;
Fig. **22D** is a further illustration of the apparatus of Fig. **22A** in which the stopper device is fully deployed.
FIG. **23** is a schematic view of an phototherapeutic apparatus for sterilization of medical instruments according to the invention;
FIG. **24** is a cross-sectional view of an optical fiber diffusive tip assembly for use in the sterilization apparatus of FIG. **23**; and
FIG. **25** is a cross-sectional view of an optical fiber and diffusive tip assembly in accordance with the present invention, further employing a disposable outer sheath.

### Detailed Description

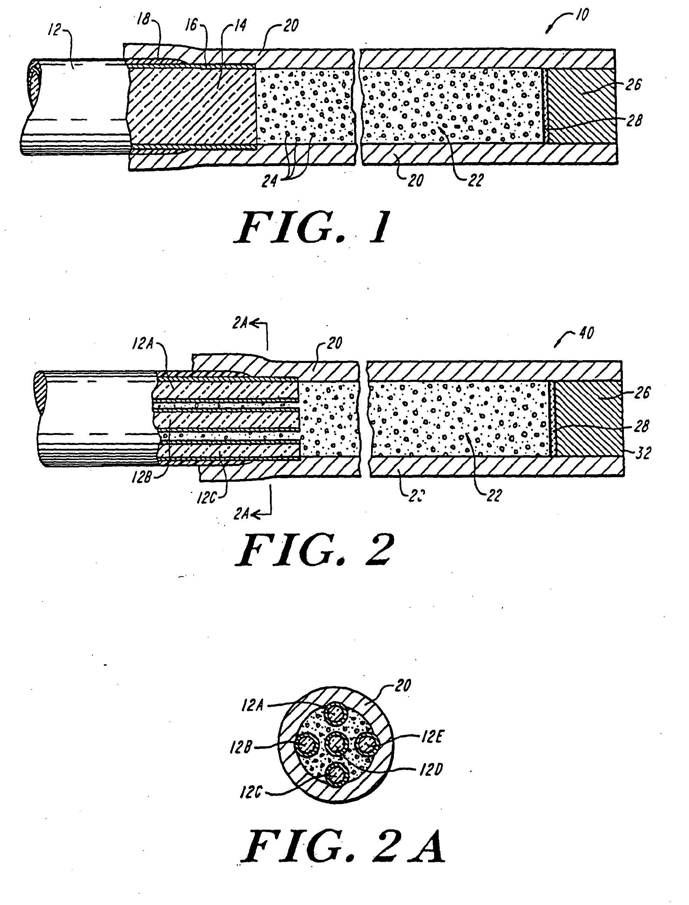

In FIG. **1** an optical fiber diffusive tip assembly **10** is shown including an optical fiber **12** having a light-transmissive core **14**, a cladding **16**, and an outer buffer coating **18**. The end face of fiber core **14** is inserted into a housing **20** which contains scattering medium **22** with individual scatterer particles **24**. Preferably, the medium **22** has a greater refractive index then the housing **20**. At the distal end of the housing **20**, an end plug **26** is disposed with a mirror reflector **28**

Light propagating through optical fiber core **14** is transmitted into the scatterer medium **22** and scattered in a cylindrical pattern along the length of the assembly **10**. Each time the light encounters a scatterer particles, it is deflected and, at some point, the net deflection exceeds the critical angle for internal reflection at the interface between the housing **20** and medium **22**. When this happens, the light will exit. Light which does not exit during this initial pass-through the tip is reflected by the mirror **28** and returned through the tip assembly. During the second pass, the remaining radiation (or at least a major portion of this returning radiation) again encounters the scatterers **22** which provide further circumferential diffusion of the light.

In FIGS. **2** and **2A**, another diffusive tip assembly **40** is shown having essentially identical elements to those shown in FIG. **1**, except for the disposition of a bundle of optical fibers **12A-12E**. The individual cores of the fibers are exposed and transmit light into the scatterer medium **22**.

FIG. **2A** is a cross-sectional view of the device of FIG. **2** showing the placement of the bundle of optical fibers **12A-12E** and the surrounding tube **20**, scatterer medium **22** and reflector **28**.

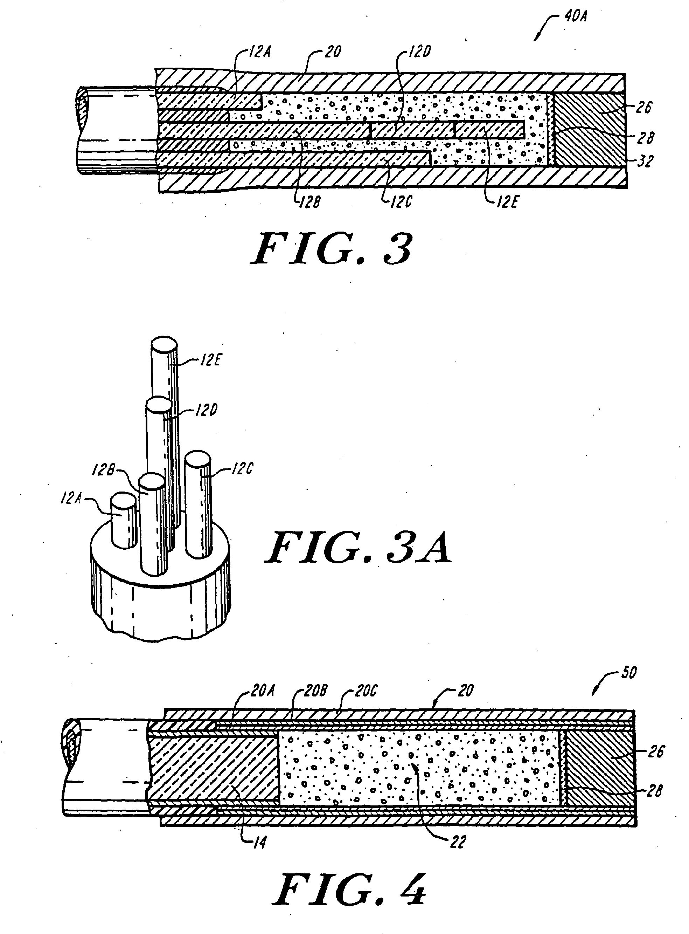

In FIGS. **3** and **3A**, another diffusive tip assembly **40A** is shown again having essentially identical elements to those shown in FIG. **1**, except for the disposition of a bundle of optical fibers **12A-12E**. The individual cores of the fibers are exposed and transmit light into the scatterer medium **22**, but the individual fibers terminate at different locations within housing **20**, thereby permitting extended axial diffusion.

FIG. **3A** is a perspective view of the fiber bundle of FIG. **3** showing the placement of the bundle of optical fibers **12A-12E** within the housing.

In FIG. **4**, an alternative diffuser tip assembly **50** is shown in which a laminate of multiple layers is used for the scatterer tube **20**. Thus, innermost layer **20A** encases the scatterer medium **22**. Surrounding this innermost layer **20A** is an intermediate layer **20B**. A third optional layer **20C** is then formed about the first two layers **20A, 20B**. Such a configuration permits the use of different polymeric tubing materials and/or allows the introduction of pigmented or etched structures as part of tubing **20**.

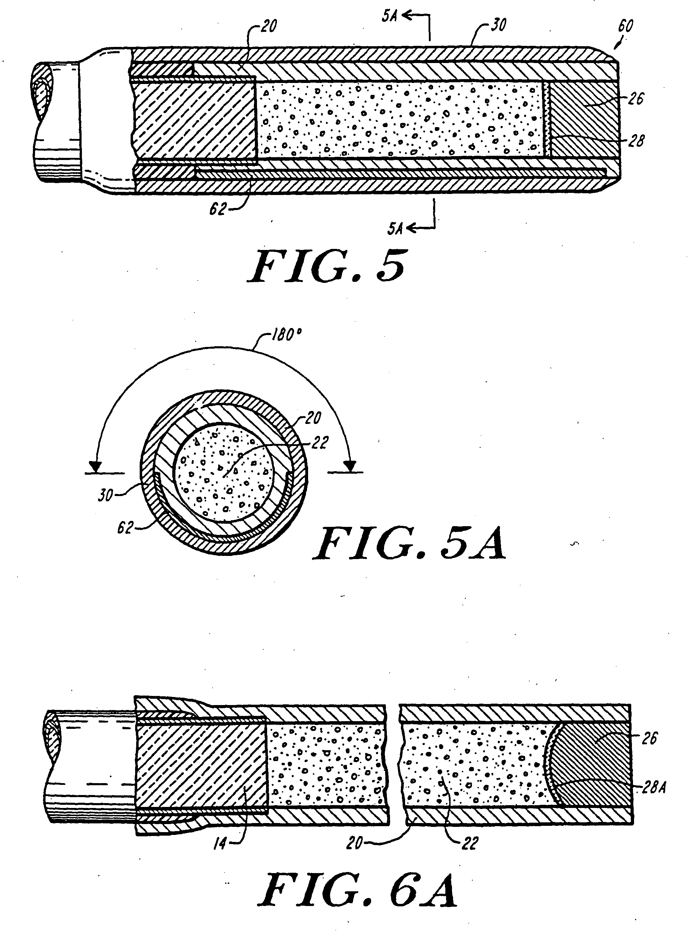

In FIG. **5**, another embodiment of a diffusing tip assembly **60** is shown incorporating a longitudinal reflector strip **62**. As further illustrated in the cross-sectional section of FIG. **5A**, the longitudinal reflector **62** can be formed as a partial layer or foil element within a laminate structure, e.g., between layer **20** and layer **30**. The longitudinal reflector **62** illustrated in FIGS. **5** and **5A** cooperates with the scatterer medium **22** to create an azimuthal exposure pattern of approximately 180°, although it should be clear that other angles of exposure can be simply achieved by widening (or narrowing) the circumferential extent of the reflector element **62**. Various alternative configurations of the reflector can be constructed. For example, the reflector can be disposed on the outside of the housing or can be formed as a coating rather than a foil element. Moreover the longitudinal reflector can be used without reflective end surface **28**, if enhanced axial uniformity is not needed.

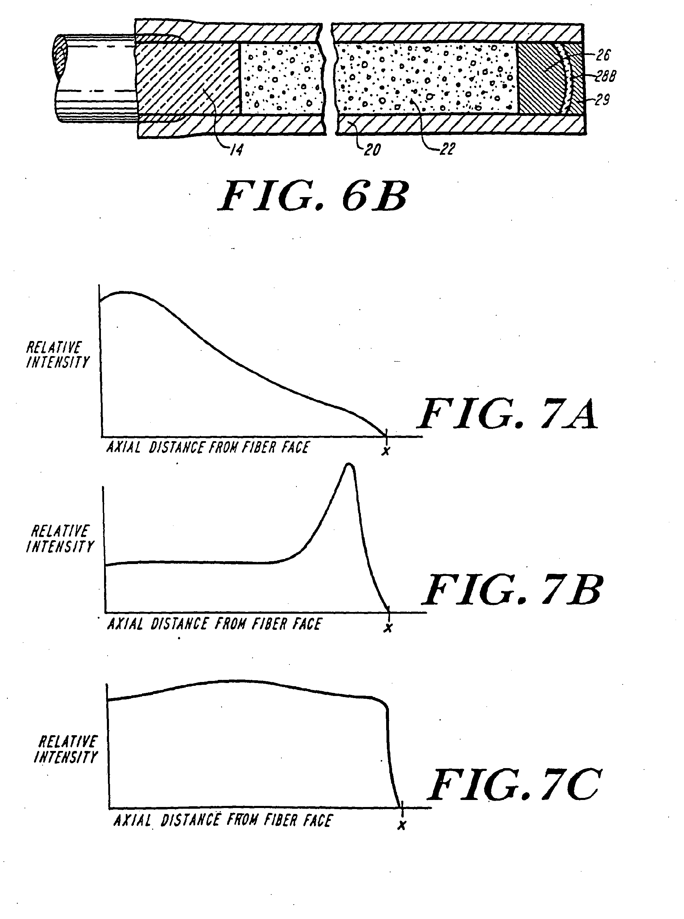

In FIG. **6A**, an alternative design is shown for the end reflector. As shown, end reflector **28A** presents a convex surface to the scattering medium and, thereby, varies the exposure pattern. In FIG. **6B**, yet another alternative design for the end reflector is shown, wherein the reflective surface is disposed at the distal rather than proximal end face of the plug **26**. In this embodiment, plug **26** is optically transmissive and the reflecting surface **28B** is formed as a concave surface. In this embodiment, a filler element **29** may also be disposed at the end of the tube **20**.

In FIGS. **7A-7C**, the effects of different scatterer concentrations on the diffusion pattern of the tip assembly is illustrated. The optimal concentration of scatterer particles incorporated into the scatterer medium will, of course, vary with the diameter of the tube, the length of the tube and the wavelength as well as other factors. Nonetheless, a optimal concentration can be readily determined empirically. FIG. **7A** illustrates the situation where too many scatterers have been loaded. Most of the light is diffused immediately upon entry into the scatterer tube. FIG. **7B** illustrates the situation where the scatterer medium is too dilute and a bright spot occurs in the vicinity of the reflector. FIG. **7C** illustrates a preferred embodiment of the present invention in which the scatterer concentration and mirror location are chosen such that the light is diffused in a substantially uniform axial pattern.

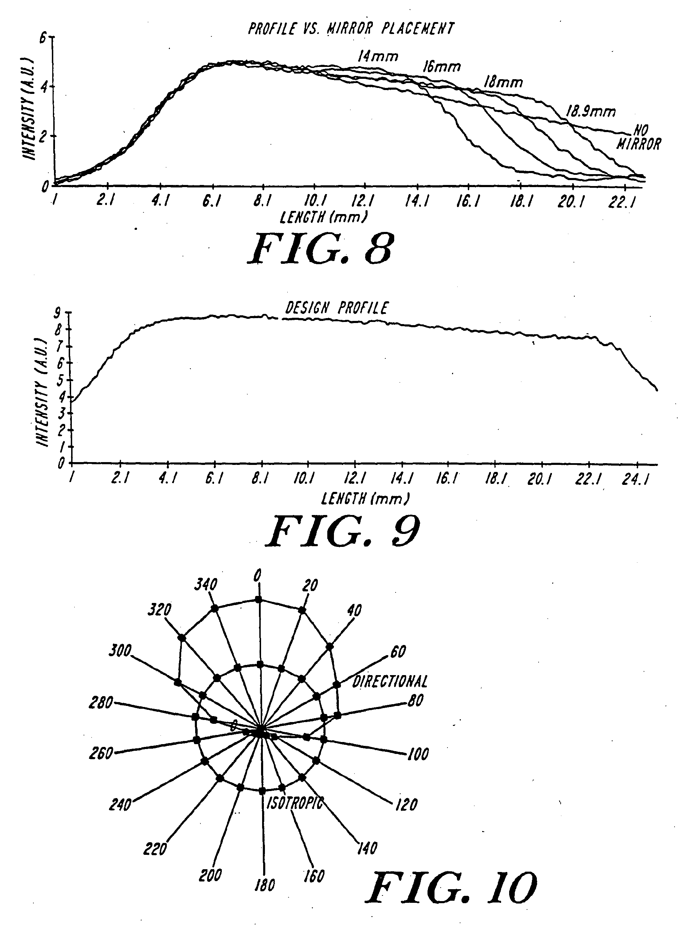

It should also be appreciated that the length of the scatterer tube (e.g., the distance between the fiber end face and the reflector) will also affect the uniformity of the diffused radiation. FIG. **8** illustrates how the mirror placement changes the exposure pattern for a given light source, tube diameter and scatterer concentration. As the tube is extended and the distance between the fiber and mirror increases, a drop-off in uniformity is observed. Again, optimal dimensions for a particular application can be determined empirically.

FIG. **9** is graph of intensity for one preferred embodiment of the invention, a fiber tip assembly similar to that shown in FIG. **1** have a Teflon® FEP tubular housing (O.D. of about 0.5 millimeters and I.D. of about 0.25 millimeters) filled with a silicone and titania scatterer composition and capped with an aluminum-coated reflective mirror. The scatterer medium was formulated by mixing 70 parts of clear silicone, Mastersil™ Formula 151-Clear (available from Masterbond, Inc. of Hackensack, New Jersey) with one part of titania filled silicone, Mastersil™ Formula 151-White (also available from Masterbond). The result was a diffusive tip assembly which uniformly transmitted red light at about 633 nanometers over its entire length of 25 millimeters.

FIG. **10** illustrated the azimuthal exposure patterns for two embodiments of the present invention. The pattern formed by the squares represents intensity of light diffused outwardly with a fiber tip assembly similar to that shown in FIG.**1**. This azimuthal exposure pattern is essentially isotropic. The pattern formed by the diamonds represents intensity of light diffused outwardly with a fiber tip assembly similar to that shown in FIG. **5**. This azimuthal exposure pattern is essentially semi-cylindrical.

An exemplary manufacturing process suitable for joining a diffuser assembly to a glass-clad or polymer-clad optical fiber having an outer diameter of about 50 to about 1000 micrometers can begin by stripping off the buffer from the end of the optical fiber, e.g., exposing about two or three millimeters of the inner fiber core and its cladding. (It is not necessary to strip the cladding away from the core.) Prior to stripping, the fiber end face preferably should be prepared and polished as known in the art to minimize boundary or interface losses. A transparent tubular structure which will form the housing for the scatterer medium is then slipped over the prepared fiber end and, preferably slid beyond the fiber end. For example, if a tip assembly of about 20 millimeters is desired, the tubing can be about 100 millimeters long and slid over about 75 millimeters of the fiber, leaving an empty lumen of about 25 millimeters in front of the fiber end face. In one preferred embodiment, the housing is Teflon® FEP tubing, available, for example, from Zeus Industries (Raritan, New Jersey).

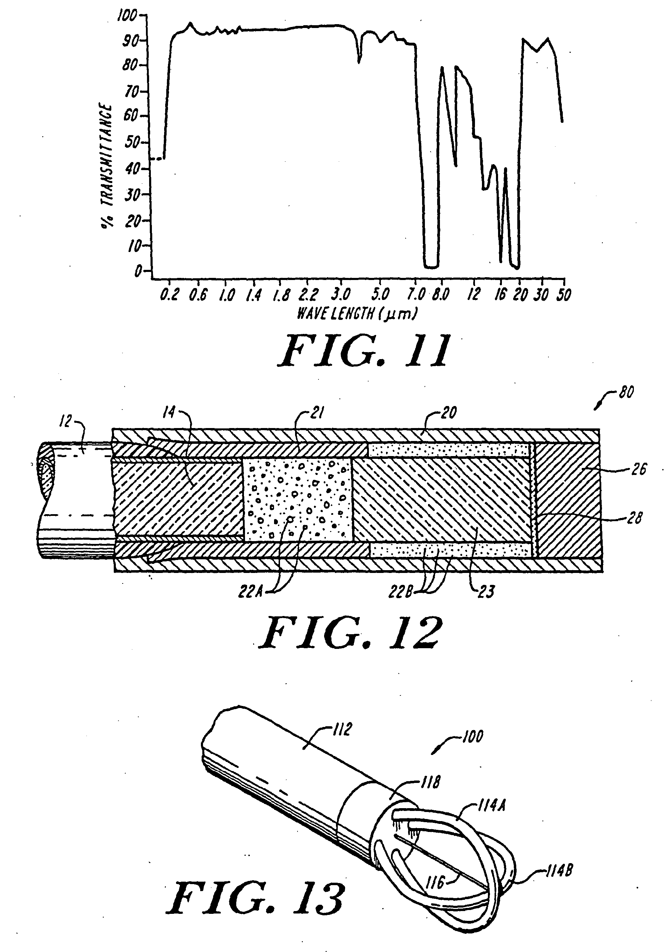

FIG. **11** illustrates the transmission spectrum of Teflon® FEP, showing that this material is well suited for use as a scatterer-encasing material across a spectrum of light from infrared to ultraviolet.

The assembly is then injected with a scatterer-loaded material, such as a silicone, epoxy or other polymeric material(if a solid diffuser is desired) or a suitable liquid, such as water or a deuterium oxide solution, containing colloidal scatterer particles, such as silica, alumina, or titania, (if a liquid diffuser is desired). As mentioned above, one exemplary scatterer medium can be formulated by mixing 70 parts of clear silicone, Mastersil™ Formula 151-Clear (available from Masterbond, Inc. of Hackensack, New Jersey) with one part of titania filled silicone, Mastersil™ Formula 151-White (also available from Masterbond), and a conventional silicone curing or hardening agent. The tube lumen should be completely filled with the silicone, epoxy or other carrier mixture to avoid entrapment of air bubbles. The reflector (e.g., an aluminum, gold or other reflector-coated plug) is inserted into the distal end of the tube. The reflector at the distal end of the scatterer tube can be a deposited metal or dielectric coating. In one preferred embodiment, a room temperature hardening agent is used and the diffuser assembly is simply allowed to solidify overnight.

Optionally, as a final step, an outer Teflon@ jacket can be disposed about the apparatus to encase and protect the entire tip assembly including the inner scatterer tube and fiber end. The outer jacket is particularly useful in constructing large azimuthal angle, non-cylindrical diffusers. In such applications, an inner scatterer assembly is constructed and then a reflective strip is disposed along the axis of the assembly to block light diffusion where the housing is covered with the reflector and thereby define a non-cylindrical exposure pattern. The extent of the circumferential coverage by the reflector will determine the azimuthal exposure pattern. The use of an outer jacket also permit a wider variety of tubing choices for the inner component of the scatterer housing. Thus, any transparent material can be used as the inner tube and the outer Teflon® jacket will still ensure that the problem of contact adhesion is minimized.

It should be clear that the manufacturing processes described above are merely illustrative, and various alternative techniques can be practiced to construct the fiber tip assemblies of the present invention. For example, automated extrusion methods and/or injection molding approaches can be employed to mass produce fibers with integral diffusive tip assemblies.

The amount of scatterer incorporated into the diffusive tip assembly will vary with the carrier and the desired length, and can therefore be adjusted to meet particular applications. Different scatterers may be more or less useful in particular applications. Table 1 below illustrates certain relevant characteristics of three different scatterer compositions:

**Table 1-**

| **Scatterer Characteristics** | | |
|---|---|---|
| **Scatterer Composition** | **Density (grams/cc)** | **Transmission Spectrum (wavelength in micrometers)** |
| TiO₂ | 4.0 | .45-11 |
| SiO₂ | 2.1 | .2-7 |
| Al₂O₃ | 3.6 | .2-9 |

In certain applications, it may be desirable to mix two or more scatterer compositions together to achieve blended characteristics.

Liquid scatterer compositions can be used to extend phototherapy into the ultraviolet (UV) and infrared (IR) regions of the spectrum. In particular, structures employing deuterium oxide and other heavy water solutions are useful to transmit IR light with low losses and minimal tip heating. Distilled water suspensions of scatterers are used for UV light delivery.

The above-described manufacturing techniques were used to produce diffusing tips joined to fibers ranging from about 100 to about 600 micrometers in diameter. When fiber bundles are joined to the diffuser tip, the individual fibers can be even smaller, e.g., as small as 25 micrometers in diameter. The cylindrical light-diffusing assemblies produced axial exposure patterns of about 2 cm to about 4 cm in length. The azimuthal exposure angle was either 360° for assemblies resembling FIG. **1** or about 180° for those resembling FIG. **5**. Other azimuthal exposure patterns can be obtained by modifying the circumferential extent of the longitudinal reflector strip **62** of FIG. **5**. The solid tubes were clear Teflon® and were injected with the above-described mixture of silicone and micron-sized titania. The liquid-filled tubes were similarly constructed but contained a water or D₂O solution loaded with colloidal alumina or silica. A exemplary liquid scatterer composition of colloidal alumina is available as Formulation 12733 from the Johnson Matthey Co. (Seabrook, New Hampshire). In use, it is preferably diluted with water by a factor of about 100:1 and pH-balanced with acetic acid.

In FIG. **12** another phototherapeutic apparatus **80** according to the invention is shown having two chambers filled with different scattering media to effect an increasing diffusion pattern. Apparatus **80** includes an optical fiber **12** having a light-transmissive core **14**. The end face of fiber core **14** is inserted into a housing **20** which contains a first chamber with a first scattering medium **21** with individual scatterer particles **22A**. The housing further includes a second chamber which can have a transparent core **23** (e.g., an FEP rod or beading) surrounded by a toroidal space filled with a second medium having scatterers **22B** of a different loading density or composition. At the distal end of the housing **20**, an end plug **26** is disposed with a mirror reflector **28**.

Light propagating through optical fiber core **14** is transmitted into the scatterer medium **22A** and scattered in a cylindrical pattern along the length of the assembly **10**. Each time the light encounters a scatterer particles, it is deflected and, at some point, the net deflection exceeds the critical angle for internal reflection at the interface between the housing **20** and medium **21**. When this happens, the light will exit. Similarly, light which passes through this first chamber is transmitted to the second chamber **23** where it encounters the scatterers **22B**, causing more of the light to be reflected. Light which does not exit during this initial pass through the tip is reflected by the mirror **28** and returned through the tip assembly. During the second pass, the remaining radiation (or at least a major portion of this returning radiation) again encounters the scatterers **22A** and **22B** which provide further circumferential diffusion of the light.

In FIG. **13**, another phototherapeutic apparatus **100** is shown including a jacket **112** having a plurality of light diffusing loops **114A, 114B** which can be expanded out of, or retracted back into, the instrument housing **112** by control wire **116**. As shown, the apparatus **100** can further include a radio opaque region **118** which facilitates location of the instrument by radiographic means. Although the apparatus is illustrated with only two loops, in some applications it can be desirable to have a greater (or lesser) number of loops.

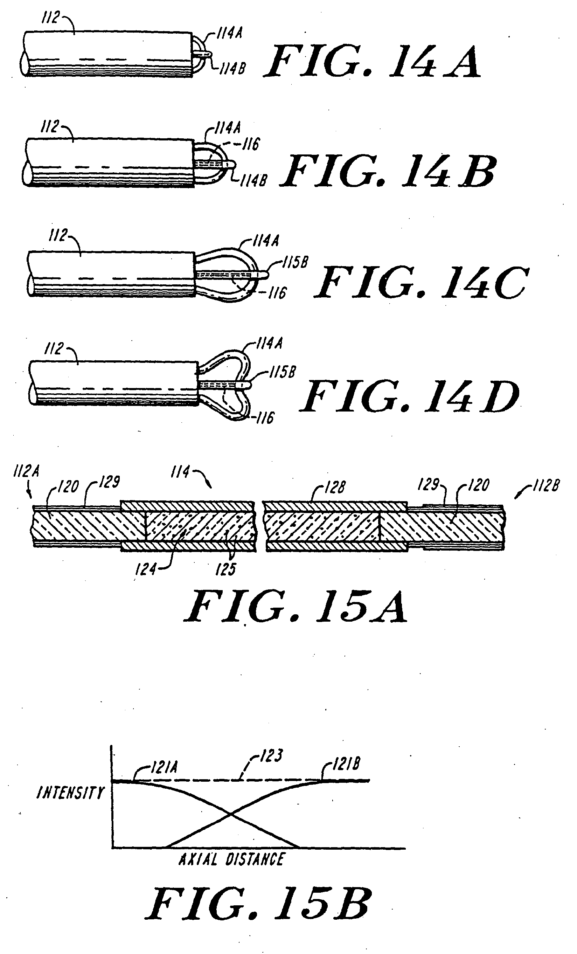

In FIGS. **14A-14D**, the deployment of loop elements **114A** and **114B** is shown schematically. FIG. **14A** illustrates a fully retracted mode in which most of the loop elements are withdrawn into the housing **112**. In FIG. **14B**, a control wire **116** has been moved partially forward and a larger portion of diffusive loop elements **114A** and **114B** projects outward from the housing **112**. In FIG. **14C**, the control wire has been slid forward even further and the loop elements **114A, 114B** now are nearly fully deployed. In FIG. **14D**, the control wire **116** is partially retracted after extension to effect a "heart-shaped" diffuser.

In FIG. **15A**, a truncated, cross-sectional view of a diffusive loop element **114** is shown connected to two optical fibers, each having a light transmissive core **120A, 120B** and a cladding/buffer coating **129**. The end face of each fiber core **120A, 120B** is inserted into a housing **128** which contains a scattering medium **124** with optional individual scatterer particles **125**. Preferably, the medium **124** has a greater refractive index than the housing **128**.

FIG. **15B** is a graph of intensity vs. radial distance for two fibers as shown in FIG. **15A**. The curve **121A** illustrates the intensity of diffused radiation vs. axial length of one fiber while curve **121B** represents a similar intensity distribution for a second fiber which has been deployed in an opposite configuration. The cumulative intensity distribution of these two fibers are shown by curve **123**. By employing pairs of fibers that are joined in opposite directions, one can thus achieve nearly uniform distribution of the diffusive radiation.

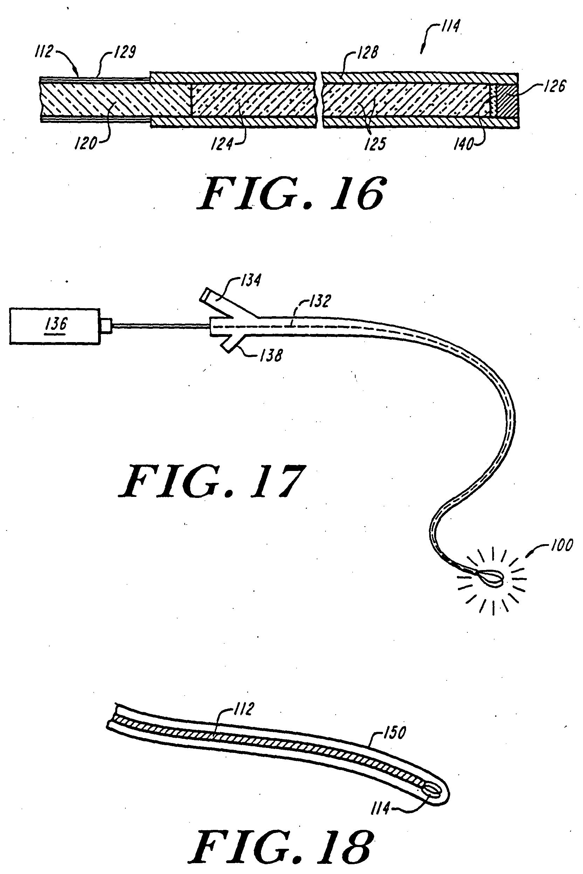

A similar radiation distribution pattern can be achieved by employing a reflective end cap on each loop, as shown in FIG. **16**. In this figure, a truncated, cross-sectional view of a diffusive loop element **114** is shown having an optical fiber with a light transmissive core **120** and a cladding/buffer coating **129**. The end face of fiber core **120** is inserted into a housing 128 which contains a scattering medium **124** with optional individual scatterer particles **125**. Preferably, the medium **124** has a greater refractive index than the housing **128**. At the distal end of the housing **128** an end plug **126** is disposed. Optionally, the end plug may also be fitted with a mirror reflector **140** to create a distribution pattern like that shown in FIG. **15B**.

Light propagating through the optical fiber core **120** is transmitted into the scatterer medium and scattered in an cylindrical pattern along the length of the assembly **114**. Each time the light encounters a scatterer particle, it is deflected and, at some point, the net deflection exceeds the critical angle for internal reflection at the interface between the housing **128** and the medium **124**. When this happens the light will exit. The housing can either be made sufficiently long to ensure that virtually all of the light entering it is eventually scattered and diffused in a single path, or as noted above, a reflective mirror can be fitted to the distal end of each diffuser assembly. When a mirror is employed, light propagating through the medium **124** will be at least partially scattered before it reaches mirror **140**. Light which does not exit during this initial pass through the tip will be reflected by mirror **140** and returned through the tip assembly. During the second pass, the remaining radiation (or at least a major portion of this returning radiation) again encounters the scatterers which provide further circumferential diffusion of the light.

In FIG. **17**, the loop diffuser apparatus of the present invention **10** is shown schematically in operation. The diffuser apparatus **100** is coupled to a source of phototherapeutic radiation **136** (e.g., a laser) and positioned within a patient's body to provide phototherapy. As shown in FIG. **17**, the diffuser assembly can be designed to fit within the instrument channel of an endoscope **132**. The endoscope can further include viewing means **134** and/or at least one additional channel **138** for the introduction of irrigation saline or therapeutic solutions. Alternatively, the diffusing assemblies of the present invention can be incorporated into catheter-type instruments that are introduced into the patient's body without the assistance of an endoscopic channel.

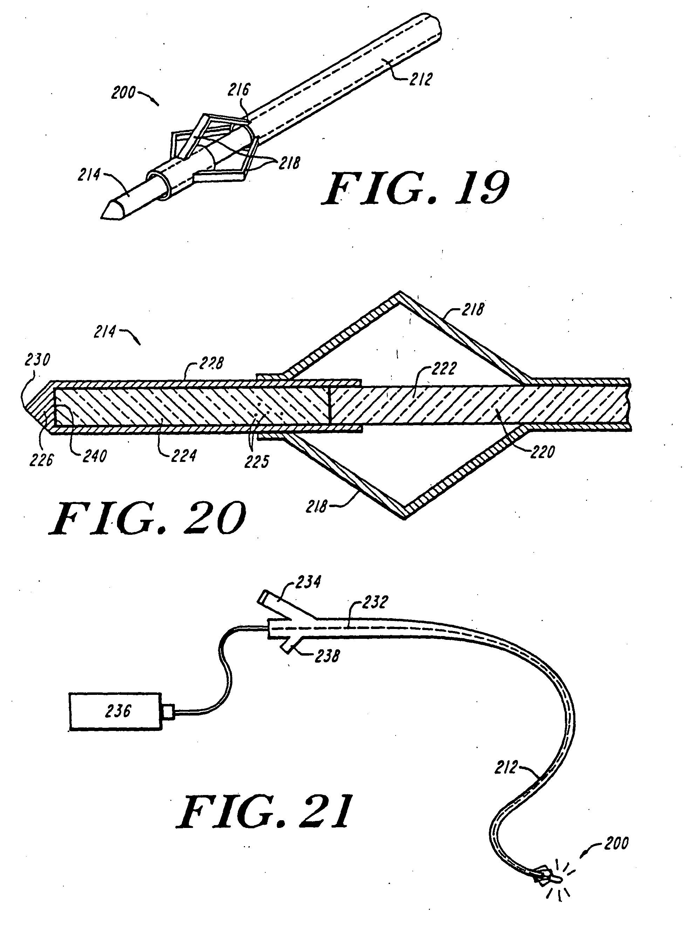

In FIG. **18**, an outer jacket (e.g., ofTeflon® material) is shown disposed about the apparatus to encase the fiber **112** and loop diffuser assembly **114**. The outer sheath surrounds the entire optical transmission apparatus and ensures that the radiation-generating components do not come into direct contact with the patient's body and, thereby, permits reuse of the instrument. Only the outer sheath **150** needs to be disposed after each use.

The devices of the present invention can be used for various therapeutic purposes. One application is photodynamic therapy (PDT), a form of light-activated chemotherapy. In this approach, photosensitive dyes are delivered by injection or other vehicles such that the dye is preferentially accumulated in cancer cells. When the cells which have taken up the dye are irradiated at an appropriate wavelength (e.g., with red light), a photochemical reaction occurs that yields radicals (usually singlet oxygen) which poison the cell. Thus, the present invention further encompasses the use of diffused radiation to activate photosensitive dyes. One advantage of the present invention is that it permits PDT at remote treatment sites via a catheter, trocar, hollow needle or other hand held instrument in a minimally invasive manner because diffusive fiber tip assemblies can now be constructed with outer diameters on the order of only a few hundred micrometers.

The present invention also encompasses the use of diffuse radiation in photocoagulation and/or hypodermic therapy of tumors and hyperplasia. For example, the phototherapy apparatuses described above can be used to treat liver, pancreatic or prostate tumors, or benign prostate hyperplasia. The application of diffuse radiation to heat prostate tissue can be used in lieu of transurethral resection of the prostate, balloon dilatation of the prostate or ultrasonic hyperthermia. In particular, the directional probes described above can be especially useful in improving the outcome of prostate treatment by heating more tissue directly in less time, and in distributing irradiation over a larger volume of prostatic tissue, thus increasing the therapeutic heating effects while reducing the risk of overheating damage to surrounding tissue structures such as the sphincter. The invention further permits interstitial laser coagulation of hepatic and pancreatic tumors. The desired effects are achieved by thermal destruction of cancerous tissue by depositing laser radiation via a diffusive fiber tip carrier by a hypodermic needle or similar instrument inserted percutaneously into the tumor. In each of these procedures, therapy can be delivered while the patient is awake; general anesthesia as well as open surgery are avoided.

In heat-based phototherapy techniques, the diffusive fiber tip assemblies of the present invention allow for the formation of large distributed heat sources within the target tissue. The invention significantly alters the rate of heat deposition in tissue, especially in the regions immediately surrounding the fiber tip, where tissue overheating and/or carbonization would limit the effectiveness and inhibit efficient heat transfer. Since the radiation is distributed by the diffuser assembly over a larger volume of tissue, more tissue is heated directly and there is less need to rely on convective or conductive heat transfer through nearby tissue to reach the periphery of the tumor.

Moreover, the materials disclosed herein for the diffusive tips and jackets further enhance the therapeutic effects by permitting high radiation transmission and low absorption, thereby ensuring the tip assembly itself does not overheat during usage. In addition, the use of Teflon® tubes and/or coatings further improve the procedures by avoiding the problem of tip fusion or contact-adhesion between the tip assembly and biological tissue during usage. It has been found that Teflon® FEP materials (polyperfluoroethylene-propylene copolymers) are preferable for most applications because they do not discolor if they are etched prior to loading with the scatterer medium, although Teflon® PFA materials (polytetrafluoroethylene polymers with perfluoroalkoxy side chains) and Teflon® PTFE (polytetrafluoroethylene) and other fluoropolmers may also be useful.

The non-cylindrical, large azimuthal angle diffusers of the present invention are also particularly useful in therapeutic applications. By directionalizing the diffused radiation, the devices disclosed herein can provide therapeutic radiation to large volumes of tissue while also protecting sensitive tissue or biological structures. For example, in prostate treatment, a semi-cylindrical or other large azimuthal angle diffuser can disposed within the urethra and rotated into a position such that the prostate is subjected to phototherapy while the patient's sphincter muscles and/or other tissue regions are largely shielded from irradiation. In addition the non-cylindrical diffusive tip assemblies can be used to deliver a greater dose of radiation to tissue and rotated, if necessary during use to effect a circumferential (or partially-circumferential scan of the target tissue at the higher intensity level.

The diffusive tip assemblies of the present invention can be used in various other medical applications, such as, for example, heat-setting of stents, activation of photoreactive suturing materials, curing of prosthetic devices, activation of adhesives for implants and the like.

In Fig. **19**, yet another phototherapeutic apparatus **200** according to the invention is illustrated having a tubular sheath **212** and an inner optically-transmissive fiber element **214**. The distal end of the sheath **212** is fluted such that axial compression of the sheath results in expansion of strut elements **218** in the fluted region **216**.

Fig **20** is a more detailed cross-sectional view of the distal end of the apparatus of Fig. **19**. The optically-transmissive element is shown having an optical fiber **220** with an optically transmissive core **222** surrounded by a cladding, and buffer coating. The end face of fiber core **222** is inserted into a housing **228** which contains a scattering medium **224** with optional individual scatterer particles **225**. Preferably, the medium **224** has a greater refractive index than the housing **228**. At the distal end of the housing **228**, and end cap **226** can be disposed. Optionally, the end cap may also be fitted with a reflective mirror **240**. The end cap **226** can further be ground or polished to a point **230** to facilitate penetration of body tissue.

Light propagating through the optical fiber core **222** is transmitted into the scatterer medium and scattered in an cylindrical pattern along the length of the assembly **214**. Again, each time the light encounters a scatterer particle, it is deflected and, at some point, the net deflection exceeds the critical angle for internal reflection at the interface between the housing **228** and the medium **224**. When this happens the light will exit. The housing can either be made sufficiently long to ensure that virtually all of the light entering it is eventually scattered and diffused in a single path, or as noted above, a reflective mirror can be fitted to the distal end of each diffuser assembly. When a mirror is employed, light propagating through the medium **224** will be at least partially scattered before it reaches mirror **240**. Light which does not exit during this initial pass through the tip will be reflected by mirror **240** and returned through the tip assembly. During the second pass, the remaining radiation (or at least a major portion of this returning radiation) again encounters the scatterers which provide further circumferential diffusion of the light:

In Fig. **21**, the phototherapeutic apparatus in the present invention **200** is shown schematically in operation. The diffuser apparatus with its fluted stopper is coupled to a source of phototherapeutic radiation **236**, (e.g., a laser) and positioned within a patient's body to provide phototherapy. As shown in Fig. **21**, the diffuser assembly can be designed to fit within a standard guiding catheter **232**. The catheter **232** can further include electrical sensing elements **234** and/or at least one additional channel **238** for introduction of saline or therapeutic solutions.

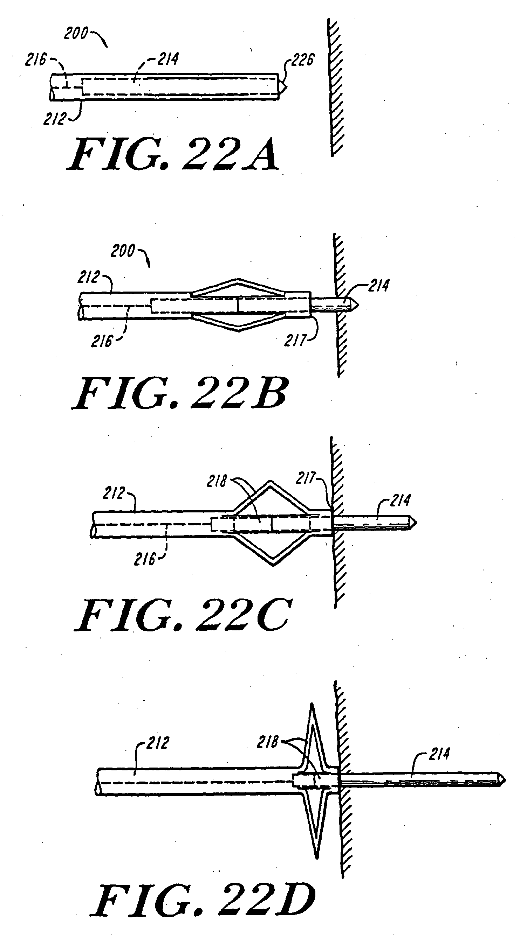

In Fig.**22A**, the use of the phototherapeutic apparatus of the present invention is shown schematically. As illustrated, the instrument **200** is positioned next to a segment of a patient's body tissue where penetration and radiation is desired. As shown, the apparatus includes an outer sheath **212** having a fluted region **216** and an inner optically-transmissive fiber element **214** with tip **226**. In one preferred embodiment, the fiber **214** and sheath **212** are constructed with sufficient clearance to permit saline or other therapeutic liquids to be released during the procedure. In particular, saline flushing of the fiber tip **214** may be desirable to cool the tissue surface proximal to the treatment site.

In Fig. **22B**, initial penetration of the apparatus **200** is shown. In this illustration, the optically-transmissive fiber has penetrated the patient's tissue but the end **217** of sheath **212** has not yet touched the tissue surface.

In Fig. **22C**, the fiber **214** has penetrated further into the patient's tissue and the sheath **212** has now been pushed into a position abutting the patient's tissue. As the instrument is advanced, the fluted region **216** begins to expand due to the compressive forces exerted during penetration. Struts **218** are pushed out radially from the body of the apparatus.

In Fig. **22D**, the apparatus is shown in a fully deployed position wherein a predetermined length of the optical fiber **214** has now penetrated the patient's body tissue and the radially-expanded struts **218** have been fully compressed into a maximal position creating a large cross-sectional obstruction to further penetration.

Various materials can be used to form the outer sheath including, for example, Teflon® and other fluorocarbon polymers. The struts **218** can be formed by axial slices at various locations on the sheath. For example to construct a four strut stopper device, one would make four longitudinal cuts into the sheath, separated by 90° from each other. The length of the cuts will determine the radial extent of the stopper. In one embodiment it may also be desirable to fill the sheath polymer with a radio-opaque substance, such as barium or bismuth in order to permit visualization under angiography.

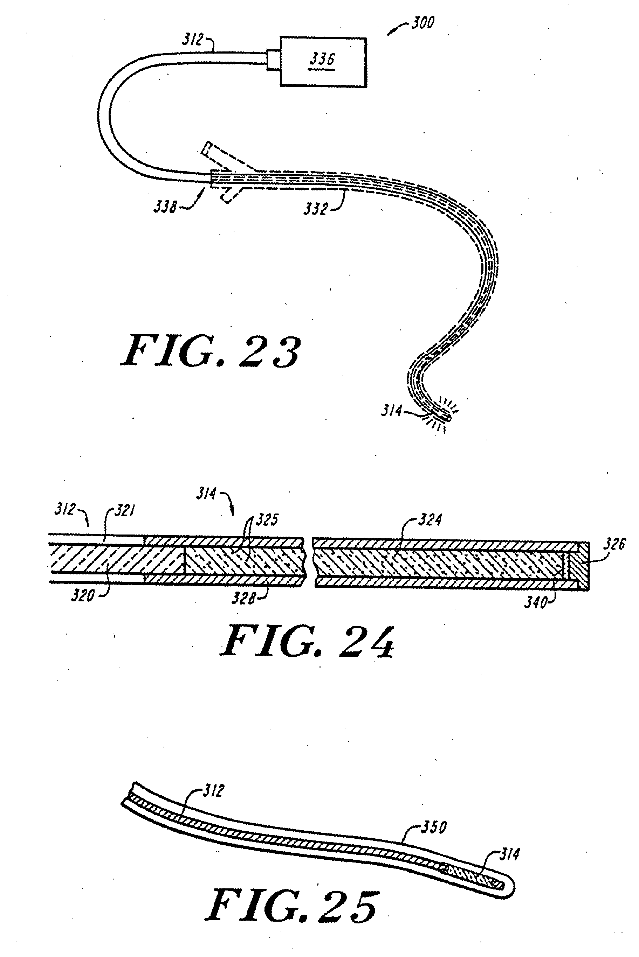

In FIG. **23** a further adaptation of the present invention is shown in a phototherapeutic apparatus **300** for sterilizing an inner lumen of an endoscopic medical instrument **332** is shown including a source of ultraviolet radiation **336**, an optical fiber **312** and a diffusive tip assembly **314**.

In use, the apparatus **300** serves to sterilize or clean an inner lumen of the endoscopic instrument **332**. The optical fiber **312** with its light-diffusing distal tip assembly **314** is inserted into the lumen requiring sterilization. In one technique, the optical fiber tip can be inserted through the entire instrument and then slowly retracted. The radiation source is activated to transmit light via the fiber **312** to the diffusive tip assembly **314**. As the apparatus is retracted through the endoscope lumen **338**, cytotoxic radiation is delivered to all portions of the inner lumen walls. Any debris or deposits on the inner lumen walls are likewise irradiated to kill any microbes which may be harbored in such deposits.

In FIG. **24** a diffusive tip assembly **314** is shown in more detail proving the optical fiber **312** having a light transmissive core **320** and a buffer coating or cladding **321**. The end face of fiber core **320** is inserted into a housing **328** which contains a scattering medium **324** with optional individual scattering particles **325**. As in previous embodiments, preferably, the medium **324** again has a greater refractive index than the housing **328**. At the distal end of the housing **328**, an end plug **326** is disposed with a mirror reflector **340**.

Light propagating through the optical fiber core **320** is transmitted into the scatterer medium **324** and scattered in a cylindrical pattern along the length of the assembly **314**. Each time the light encounters a scatterer particle, it is deflected and, at some point, the net deflection exceeds the critical angle for internal reflectance at the interface between the housing **328** and the medium **324**. When this happens, the light will exit. Light which does not exit during the initial pass through the tip is reflected by the mirror **328** and returned through the tip assembly. During the second pass, the remaining radiation (or at least the major portion of this returning radiation) again encounters the scatterers **325** which provide further circumferential diffusion of the ultraviolet light.

The optimal concentration of scatterer particles incorporated into this scatterer medium will, of course, vary with the diameter of the tube, the length of the tube and the wavelength as well as other factors. Nonetheless, an optimal concentration can readily be determined empirically for ultraviolet radiation in the range of about 400 nanometers to about 200 nanometers, one preferred composition for the scatterer medium is colloidal alumina suspended in acetic acid. It should also be appreciated that the length of the scatterer tube (e.g., the distance between the fiber end facing and the reflector) will also affect the uniformity of the diffused radiation.

Optionally, as shown in FIG. **24**, an outer Teflon® jacket **350** can be disposed about the apparatus as a final step to encase and protect the entire tip assembly including the inner scatterer tube **314** and fiber end **312**.

In use, the apparatus is slid into an endoscope lumen and connected to a UV light source. The light source is activated and the UV radiation is transmitted to the diffusive tip, where the scatterers project a cylindrical exposure pattern to the lumen walls. The apparatus can then be slid forward or backwards (or in both directions) to bathe the entire lumen with sterilizing irradiation.

## Claims

1. A diffusive tip assembly for use with an optical fiber (**12**) for diffusion of radiation propagating through said fiber, the tip assembly comprising:
a light transmissive housing (**20**) having a first end adapted to receive a light transmitting optical fiber (**12**) and a second end with a reflective surface, and a light scattering medium (**22**) disposed within the housing, wherein radiation propagating through a fiber when connected to said assembly enters the scattering medium and a portion of the radiation is scattered outward through said housing, and another portion passes through the scattering medium and is reflected by the reflective surface for retransmission through said scattering medium,
**characterized in that** the reflective end surface (**28**) further comprises a dielectric coating.

2. A diffusive tip assembly for use with an optical fiber (**12**) for diffusion of radiation propagating through said fiber, the tip assembly comprising:
a light transmissive housing (**20**) having a first end adapted to receive a light transmitting optical fiber (**12**) and a second end with a reflective surface, and a light scattering medium (**22**) disposed within the housing, wherein radiation propagating through a fiber when connected to said assembly enters the scattering medium and a portion of the radiation is scattered outward through said housing, and another portion passes through the scattering medium and is reflected by the reflective surface for retransmission through said scattering medium,
**characterized by** a longitudinal reflector strip (**62**) provided in the housing (**20**) to control the azimuthal extent of the light emitted from the tip

3. The assembly of claim 1 or 2, including a light scattering medium (**22**) with scattering particles (**24**) uniformly dispersed therein, wherein that the concentration of light scattering particles in the scattering medium and the position of the reflective end surface (**28**) within the housing (**20**) are selected such that the light portions emitted during the initial and reflected paths are complementary to each other and result in a substantially uniform axial distribution of radiation over the length of the tip apparatus.

4. A method of manufacturing a light diffusive tip assembly comprising:
providing an elongate, light transmissive, housing (**20**) having a first end adapted to receive a light transmitting optical fiber (**12**) and a second end adapted to receive a reflective end surface (**28**),
selecting a light scattering medium (**22**) having a uniform concentration of scattering particles (**24**) to be disposed within the housing (**20**), and
assembling the scattering medium (**22**) within the housing (**20**) and joining the fiber (12) to the first end and the end surface to the second end of the housing, such radiation propagating through a fiber when connected to said assembly enters the scattering medium,
**characterized in that** the concentration of scattering particles (**24**) and the length of the housing (**20**) are chosen such that a portion of the radiation is scattered outward through said housing during an initial path, and another portion passes through the scattering medium (**22**) and is reflected by the reflective end surface (**28**) and is also scattered outward during a reflected path through said scattering medium, and the light portions emitted during the initial and reflected paths are complementary to one another to provide a substantially uniform axial distribution of radiation over the length of the tip assembly in use.

5. The method of claim 4, wherein the method further comprises incorporating a longitudinal reflector strip (**62**) into the housing (**20**) to control the azimuthal extent of the light emitted from the tip.

6. The method of claim 4, wherein the step of selecting a light scattering medium (**22**) further comprises selecting a light scattering medium having a greater refractive index than the housing (**20**).

7. The method of claim 4, wherein the step selecting a light scattering medium (**22**) further comprises selecting a light scattering medium of silicone or epoxy.

8. The method of claim 7, wherein the step of selecting a light scattering medium (**22**) further comprises selecting a light scattering medium of silicone.

9. The method of claim 8, wherein the method further comprises the step of adding a hardening or curing agent to said light scattering medium (**22**).

10. The method of any one of the preceding claims, wherein the step of selecting a light scattering medium (**22**) further comprises selecting a light scattering medium having a uniform concentration of silica, alumina or titania as scattering particles (**24**).

11. The method of claim 4, wherein the step assembling further comprises injecting the assembly with the scattering medium (**22**).

12. The method of claim 4, wherein the step of selecting a scattering medium (**22**) further comprises selecting from the group of a water or deuterium oxide solution containing colloidal scattering particles (**24**).

13. The method of claim 12, wherein the colloidal scattering particles (**24**) are selected from the group consisting of silica, alumina or titania.

14. The method of claim 12, further comprising the step of balancing the pH of the water or a deuterium oxide solution using acetic acid.

15. The method of claim 4, further comprising the step of providing a dielectric coating as the reflective end surface (**28**).
